# EUROPÄISCHE PATENTSCHRIFT

(11) **EP 0 665 849 B1**
(45) Veröffentlichungstag und Bekanntmachung des Hinweises auf die Patenterteilung: **27.08.1997**
(21) Anmeldenummer: 93923513.1
(22) Anmeldetag: 21.10.1993
(51) Int. Cl.: C07J 1/00

(54) **VERFAHREN ZUM ALKYLIEREN VON ÖSTRONDERIVATEN**
METHOD OF ALKYLATING OESTRONE DERIVATIVES
PROCEDE D'ALKYLATION DE DERIVES D'ESTRONES

(30) Priorität: 22.10.1992 DE 4235657
(43) Veröffentlichungstag der Anmeldung: 09.08.1995
(73) Patentinhaber: SCHERING AKTIENGESELLSCHAFT, 13342 Berlin (DE)
(72) Erfinder: SEILZ, Carsten, D-59174 Kamen (DE); HÜBL, Dieter, D-59368 Werne (DE)
(86) Internationale Anmeldenummer: EP9302905
(87) Internationale Veröffentlichungsnummer: WO9409024

(56) Entgegenhaltungen:
- TETRAHEDRON, (INCL. TETRAHEDRON REPORTS) Bd. 46, Nr. 6 , 1990 , OXFORD GB Seiten 1839 - 1848 G. BARCELO ET AL 'Pentaalkylguanidines as Etherfication and Esterification Catalysts' in der Anmeldung erwähnt
- SYNTHESIS. Nr. 5 , Mai 1986 , STUTTGART DE Seiten 382 - 383 M. LISSEL ET AL 'Dimethylcarbonat als Methylierungsmittel unter Phasen-Transfer-Katalytischen Bedingungen'

## Beschreibung

Östronderivate sind bekannte Ausgangsverbindungen für die Synthese von einer Vielzahl von Folgeprodukten wie beispielsweise 19-Nor-Verbindungen. Dabei ist es häufig unerläßlich, die Hydroxylgruppen zu alkylieren.

Dieses erfolgt üblicherweise mit den bekannten klassischen Alkylierungsmitteln wie insbesondere Dialkylsulfaten. Mit diesen Reagenzien werden unter milden Reaktionsbedingungen in der Regel hohe Ausbeuten erzielt. Aufgrund ihrer guten Alkylierungseigenschaften wirken sie jedoch schon in kleinsten Konzentrationen ausgesprochen kanzerogen, mutagen und teratogen. Dieses Potential macht eine äußerst sorgfältige Aufbereitung sowohl der Syntheseprodukte als auch der zu entsorgenden Produktionshilfsmittel erforderlich.

Es bestand daher in der betrieblichen und labortechnischen Praxis ein dringender Bedarf für ein Verfahren, welches die gewünschten Etherbildungen in hohen Ausbeuten, aber ohne die nachteiligen toxischen Nebenwirkungen ermöglicht.

Aus der Literatur ist die Verwendung von Kohlensäureestern bekannt, wobei diese beispielsweise unter Phasentransferbedingungen oder in Gegenwart von Phosphinen oder basischen Katalysatoren unter mehr oder minder drastischen Bedingungen umgesetzt werden.

So ist aus Tetrahydron, 1990, Vol. 46, Seiten 1839 - 1848 bekannt, Kohlensäureester in Gegenwart von Pentaalkylguanidinen als Katalysatoren einzusetzen.

Hohe Ausbeuten wurden dabei an Aminen, Merkaptanen und einfachen Alkoholen oder Phenolen erzielt.

Die Veretherung von Östronderivaten unter den dort beschriebenen Bedingungen führt nur zu unzureichenden Umsetzungen, in der Regel < 90 %.

Aufgabe der vorliegenden Erfindung war es daher, ein Verfahren zu finden, mit welchem Östronderivate ohne Mitverwendung von toxikologisch bedenklichen Reagentien in hohen Ausbeuten umgesetzt werden können.

Gegenstand der Erfindung ist ein Verfahren zum Alkylieren von Östronderivaten, welches dadurch gekennzeichnet ist, daß in erster Stufe eine 5 - 20, vorzugsweise 8 - 15 Gew.- %ige Suspension der Östronderivate in Dimethylformamid hergestellt und darin 1 - 5, vorzugsweise 2 - 4 Mol Kohlensäurediester pro Mol zu alkylierende OH-Gruppen sowie 1 - 10, vorzugsweise 3 - 7 Mol-% eines Guanidins und/oder eines Alkylguanidins, bezogen auf Östronderivat, gelöst und in
zweiter Stufe aus der Reaktionsmischung der darin enthaltende Sauerstoff weitgehend entfernt und in
dritter Stufe die Reaktionstemperatur durch kontinuierliches Erwärmen auf 100 - 200 °C, vorzugsweise 130 - 170 °C, eingestellt und die Reaktion unter dem sich einstellenden Systemdruck innerhalb von 3 - 36 h durchgeführt wird.

Ein weiterer Gegenstand der Erfindung ist dadurch gekennzeichnet, daß als Dialkylcarbonate Dimethylcarbonat, Diethylcarbonat, Di-n-propyl- und Di-i-propylcarbonat, Di-prim. (sek. tert.)-Butylcarbonat mitverwendet werden.

Ein weiterer Gegenstand der Erfindung ist dadurch gekennzeichnet, daß als Katalysator Guanidine selbst oder mit Methyl-, Ethyl-, n-Propyl, i-Propyl, prim. (sek. tert.)-Butyl-Resten verwendet werden.

Mit dem erfindungsgemäßen Verfahren können Östronderivate der Art alkyliert werden, welche in der Regel überwiegend aus Verbindungen der allgemeinen Formel 1 a 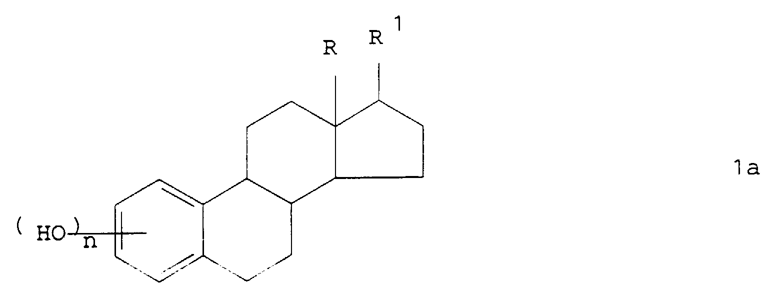 bestehen und daneben noch Verbindungen der allgemeinen Formel 1 b 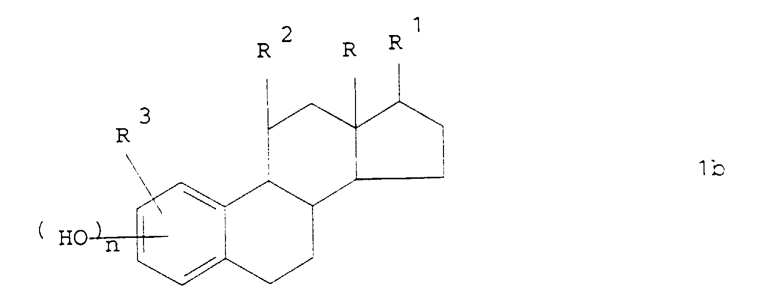 und/oder der allgemeinen Formel 1 c 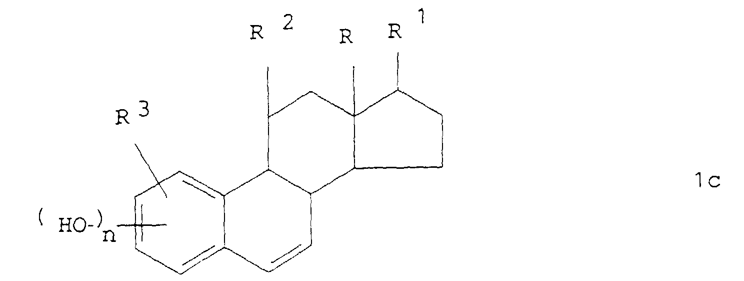 enthalten, worin R = -CH₃, -C₂ H₅, R¹ = HO- oder O=, R² und R³ unabhängnig voneinander OH- oder R bedeuten und n = 1 oder 2 ist.

Erfindungsgemäß bevorzugt werden Verbindungen der allgemeinen Formel 1a bzw. deren technische Mischungen verwendet, in welchen R = -CH₃, R¹ die Gruppe O= und n = 1 ist.

Diese Ostronderivate werden als 5 - 20 Gew.-%ige, vorzugsweise 8 - 15 Gew.-%ige Suspension in Dimethylformamid eingesetzt.

Als Alkylierungsreagentien werden hierfür Kohlensäurediester der allgemeinen Formel (2) 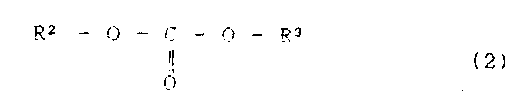 eingesetzt, worin R² und R³ geradkettige oder verzweigte Kohlenwasserstoffreste mit 1 - 10 C-Atomen, vorzugsweise 1 - 4 C-Atomen, gegebenenfalls substituierte cycloaliphatische, alicyclische, araliphatische Kohlenwasserstoffreste mit 5 - 10 C-Atomen, vorzugsweise 6 - 8 C-Atomen, sein können. Erfindungsgemäß bevorzugt sind Kohlensäuredimethylester, Kohlensäurediethylester, Kohlensäuredipropylester, Kohlensäuredi-n-butylester und Kohlensäuredi-i-butylester. Pro Mol zu alkylierende OH-Gruppe des Östronderivates werden 1 - 5 Mol, vorzugsweise 2 - 4 Mol, des Kohlensäurediesters eingesetzt.

Die Wahl der jeweiligen Kohlenwasserstoffreste R², R² bestimmt sich einmal durch die angestrebten Reaktionsbedingungen wie Systemdruck (Überdruck, Normaldruck), Reaktionstemperaturen und Reaktionszeiten, zum anderen aber auch durch die Art der weiteren Synthesestufen des veretherten Östronderivates.

Die erfindungsgemäß mitverwendeten Katalysatoren sind Guanidine der allgemeinen Formel (3) 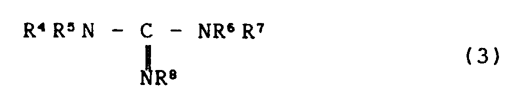 worin R⁴, R⁵, R⁶, R⁷, R⁸ Wasserstoffatome sein oder die Bedeutung von R², R³ haben können.

Sind ein oder mehrere der Reste R⁴ - R⁸ Wasserstoffatome, werden diese unter den Reaktionsbedingungen der dritten Stufe durch die Reste R², R³ des Kohlensäurediesters substituiert, wobei letztlich das pentasubstituierte Guanidinderivat als die katalytisch aktive Verbindung gilt.

Erfindungsgemäß bevorzugt werden die handelsüblichen Alkylderivate mit einer Kettenlänge von 1 - 4 C-Atomen, insbesondere die Methyl- und Ethylguanidine in Mengen von 1 - 10, vorzugsweise 3 - 7 Mol-%, bezogen auf Ostronderivat, eingesetzt.

Das erfindungsgemäße Verfahren wird bei niedrigsiedenden Kohlensäurediestern vorteilhafterweise in einem Druckreaktor durchgeführt. Bei höhersiedenden Diestern mit Siedepunkten ab ca. 100 °C , vorteilhafterweise im Bereich von ca. 130 - 170 °C, kann das Verfahren sowohl unter Rückflußbedingungen als auch unter erhöhtem Druck druchgeführt werden.

Hierbei wird in erster Stufe eine 5 - 20 Gew.-%ige, vorzugsweise 8 - 15 Gew.-%ige Suspension der Östronderivate in Dimethylformamid hergestellt und darin 1 - 5 Mol, vorzugsweise 2 - 4 Mol Kohlensäurediester, bezogen auf zu alkylierende OH-Gruppen sowie 1 - 10 Mol-%, vorzugsweise 3 - 7 Mol-%, bezogen auf das eingesetzte Östronderivat, eines Guanidins und/oder eines Alkylguanidins gelöst. Diese Reihenfolge ist nicht kritisch und kann entsprechend den Vorgaben der betrieblichen Praxis variiert werden.

In der zweiten Stufe wird aus der Reaktionsmischung der darin enthaltene Sauerstoff weitgehend entfernt.

Hierzu wird aus dem Reaktor in praxisüblichen Verfahren durch mehrmaliges Evakuieren und Belüften mit Inertgasen wie vorzugsweise Stickstoff weitestgehend der gelöste Sauerstoff entfernt.

In dritter Stufe wird die Reaktionsmischung kontinuierlich auf 100 - 200 °C, vorzugsweise 130 - 170 °C, erwärmt und diese Temperatur bei dem sich einstellenden Systemdruck 3 - 36 h gehalten.

Der Systemdruck kann innerhalb weiter Grenzen variiert werden und hängt im wesentlichen von der Art des Alkylierungsmittels und dem Füllstand des Reaktors ab.

Wird das Verfahren unter Rückflußbedingungen durchgeführt, wird die Inertisierung durch Überlagerung mit Inertgas aufrechterhalten.

Die Reaktionszeiten sind außer von der Art der eingesetzten Edukte wesentlich abhängig von der Höhe der gewählten Reaktionstemperatur. Im erfindungsgemäß bevorzugten Bereich von 130 - 170 °C liegen sie im Falle von Östron und Dimethylcarbonat im Bereich von 3 - 24 Stunden.

Die Aufarbeitung der Reaktionsprodukte wird mit den auf diesem Gebiet üblichen Verfahren durchgeführt.

### Beispiel 1:

### Erfindungsgemäß:

15,0 g (55 mMol) Estron (Gehalt: 99,5 Gew.-%), 13,8 ml (164 mMol) Dimethylcarbonat und 0,34 ml (2,5 mMol) Tetramethylguanidin werden in 100 ml Dimethylformamid (DMF) bei Raumtemperatur suspendiert.

Die vorbereitete Reaktionsmischung wird auf 40 °C erwärmt, wobei sich eine klare Lösung bildet und dann ca. 30 min. im Ultraschallbad durch Stickstoffzuführung unter die Lösungsmitteloberfläche inertisiert.

Die Lösung wird in einen Autoklaven überführt und für 24 h auf 130 °C unter Rühren erwärmt.

Die entstandene Suspension wird mit 1000 ml Wasser versetzt und der Niederschlag abgesaugt.

Man erhält 15,1 g (100 % v. E. - 53 mMol - 96 % d. Th.) des gewünschten Estronmethylethers (Gehalt: 99 % (HPLC, ext. Standard)).

### Beispiel 2:

Beispiel 2 wurde analog Beispiel 1 durchgeführt, mit der Abänderung, daß ein Estron mit einem Gehalt von 95 Gew.-% zur Alkylierung eingesetzt wurde.

Man erhält den gewünschten Estronmethylether mit einem Gehalt von 95 Gew.-% bei einer Ausbeute von 15 g.

### Beispiel 3:

Durchführung der Alkylierung gemäß Beispiel 1, mit der Abänderung, daß die Reaktion bei 170 °C durchgeführt wurde und nach 3 h beendet war.

Der gewünschte Estronmethylether fällt in einer Reinheit von 99,3 Gew.-% an, bei einer Ausbeute von 15,1 g.

### Beispiel 4 (Vergleich):

Die Alkylierung erfolgte gemäß Beispiel 3, mit der Abänderung, daß der in der Reaktionsmischung enthaltene Sauerstoff nicht gemäß erfindungsgemäßem Verfahren Stufe 2 entfernt, sondern die Reaktion lediglich in Stickstoffatmosphäre durchgeführt wurde.

Man erhält mit einer Ausbeute von 97 % v. E. den gewünschten Estronmethylether in einer Reinheit von 90 Gew.-%.

### Beispiel 5 (Vergleich)

Die Durchführung der Alkylierung erfolgte analog Beispiel 3, mit der Abänderung, daß anstelle von Tetramethylguanidin 9,2 g (112 mMol) Kaliumtertiärbutylat eingesetzt werden.

Man erhält in einer Ausbeute von 101 % v. E. den gewünschten Estronmethylether in einer Reinheit von lediglich 57 Gew.-%.

### Beispiel 6 (Vergleich):

Die Durchführung der Alkylierung erfolgte analog Beispiel 1, mit der Abänderung, daß anstelle von Dimethylformamid gleiche Mengen an
a) Tetrahydrofuran
b) Butylglykolacetat
eingesetzt werden.

Man erhält in einer Ausbeute von 100 % v. E. einen weißen Niederschlag, in welchen neben dem nicht umgesetzten Estron der gewünschte Methylether mit Gehalten von
a) < 30 Gew.-%
b) < 25 Gew.-%
enthalten war.

## Patentansprüche

1. Verfahren zum Alkylieren von Östronderivaten, dadurch gekennzeichnet, daß in erster Stufe eine 5 - 20, vorzugsweise 8 - 15 Gew.-%ige Suspension der Östronderivate in Dimethylformamid hergestellt und darin 1 - 5, vorzugsweise 2 - 4 Mol Kohlensäurediester pro Mol zu alkylierende OH-Gruppen sowie 1 - 10, vorzugsweise 3 - 7 Mol-% eines Guanidins und/oder eines Alkylguanidins, bezogen auf Östronderivat, gelöst und in zweiter Stufe aus der Reaktionsmischung der darin enthaltende Sauerstoff weitgehend entfernt und in dritter Stufe die Reaktionstemperatur durch kontinuierliches Erwärmen auf 100 - 200 °C, vorzugsweise 130 - 170 °C, eingestellt und die Reaktion unter dem sich einstellenden Systemdruck innerhalb von 3 - 36 h durchgeführt wird.

2. Verfahren gemäß Anspruch 1, dadurch gekennzeichnet, daß als Dialkylcarbonate Dimethylcarbonat, Diethylcarbonat, Di-n-propyl- und Di-i-propylcarbonat, Di-prim. (sek. tert.)-Butylcarbonat mitverwendet werden.

3. Verfahren gemäß den Ansprüchen 1 und 2, dadurch gekennzeichnet, daß als Katalysator Guanidine selbst oder mit Methyl-, Ethyl-, n-Propyl, i-Propyl, prim. (sek. tert.)-Butyl-Resten verwendet werden.

## Claims

1. Process for the alkylation of oestrone derivatives, characterised in that, in a first step, a from 5 to 20% by weight, preferably from 8 to 15% by weight, suspension of the oestrone derivatives in dimethylformamide is prepared and there are dissolved therein from 1 to 5 mol, preferably from 2 to 4 mol, of carbonic acid diester per mol of OH groups to be alkylated, as well as from 1 to 10 mol %, preferably from 3 to 7 mol %, based on the oestrone derivative, of a guanidine and/or an alkylguanidine, and, in a second step, the oxygen contained in the reaction mixture is largely removed therefrom, and, in a third step, the reaction temperature is set by continuous heating at from 100 to 200°C, preferably from 130 to 170°C, and the reaction is carried out under the ensuing system pressure within a period of from 3 to 36 hours.

2. Process according to claim 1, characterised in that there are used as dialkyl carbonates dimethyl carbonate, diethyl carbonate, di-n-propyi carbonate, diisopropyl carbonate, di-prim. (sec. tert.)-butyl carbonate.

3. Process according to claims 1 and 2, characterised in that there are used as catalyst guanidine itself or guanidines with methyl, ethyl, n-propyl, isopropyl, prim. (sec. tert.)-butyl radicals.

## Revendications

1. Procédé pour l'alkylation des dérivés d'estrones, caractérisé en ce que l'on prépare dans une première étape une suspension de 5 à 20, de préférence à 8 à 15 % en poids des dérivés d'estrones dans du diméthylformamide et on dissout dans cette suspension 1 à 5, de préférence 2 à 4 moles d'un diester d'acide carbonique par mole de groupe OH à alkyler, ainsi que de 1 à 10, de préférence de 3 à 7 % en moles d'une guanidine et/ou d'une alkylguanidine par rapport au dérivé d'estrone, et dans une deuxième étape on élimine l'oxygène essentiellement contenu dans le mélange réactionnel et dans une troisième étape, on établit la température réactionnelle par chauffage en continu à 100 à 200 °C, de préférence de 130 à 170 °C, et on conduit la réaction pendant 3 à 36 heures sous la pression établie du système.

2. Procédé selon la revendication 1, caractérisé en ce que l'on utilise comme carbonates de dialkyles le carbonate de diméthyle, le carbonate de diéthyle, le carbonate de di-n-propyle et de di-i-propyle, le carbonate de di-prim. (sec., tert.)-butyle.

3. Procédé selon les revendications 1 et 2, caractérisé en ce que l'on utilise en tant que catalyseurs les guanidines elles-mêmes ou les guanidines portant des restes méthyle, éthyle, n-propyle, i-propyle, prim. (sec., tert.)-butyle.
